(19) Europäisches Patentamt
European Patent Office
Office européen des brevets

(11) Veröffentlichungsnummer: **0 090 204**
**A1**

(12) **EUROPÄISCHE PATENTANMELDUNG**

(21) Anmeldenummer: **83102204.1**

(22) Anmeldetag: **07.03.83**

(51) Int. Cl.³: **C 07 C 79/46**, C 07 C 76/00

(30) Priorität: **19.03.82 DE 3210056**

(43) Veröffentlichungstag der Anmeldung: **05.10.83**
**Patentblatt 83/40**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Cölln, Reimer, Dr., In den Birken 67, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**

(54) **Optisch aktive, linksdrehende Phenoxybenzoesäure-Derivate, Verfahren zu ihrer Herstellung und ihre Verwendung als Herbizide.**

(57) Neue linksdrehende Enantiomere von Phenoxybenzoesäure-Derivaten der Formel

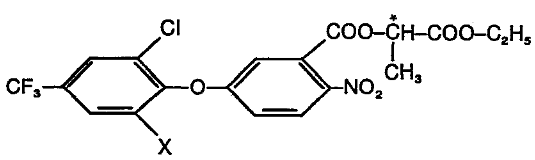

(I)

in welcher
X für Wasserstoff oder Chlor steht, ein Verfahren zur Herstellung dieser Stoffe und deren Verwendung zur Bekämpfung von Unkräutern.

EP 0 090 204 A1

BAYER AKTIENGESELLSCHAFT 5090 Leverkusen, Bayerwerk

Zentralbereich
Patente, Marken und Lizenzen Dü/AB/bo/c
Ia

# Optisch aktive, linksdrehende Phenoxybenzoesäure-Derivate, Verfahren zu ihrer Herstellung und ihre Verwendung als Herbizide

Die Erfindung betrifft neue linksdrehende Enantiomere*) von Phenoxybenzoesäure-Derivaten, ein Verfahren zu ihrer Herstellung und ihre Verwendung als Herbizide.

Es ist bereits bekannt, daß Racemate der Verbindungen 5-(2-Chlor-4-trifluormethyl-phenoxy)-2-nitro-benzoesäure-(1-ethoxycarbonylethyl)-ester und 5-(2,6-Dichlor-4-trifluormethyl-phenoxy)-2-nitro-benzoesäure-(1-ethoxycarbonyl-ethyl)-ester zur Unkrautbekämpfung eingesetzt werden können (vgl. DE-OS 29 50 401 und DE-OS 30 29 728). Die Wirksamkeit dieser Stoffe ist gut, jedoch sind die selektiven herbiziden Eigenschaften nicht immer voll befriedigend.

---

*) Unter linksdrehenden Enantiomeren sind hier jeweils diejenigen optisch aktiven Verbindungen zu verstehen, welche die Schwingungsebene von linear polarisiertem Licht nach links drehen. Diese Verbindungen besitzen im vorliegenden Fall am asymmetrisch substituierten Kohlenstoffatom die R-Konfiguration.

Es wurden jetzt die linksdrehenden Enantiomeren der Phenoxybenzoesäure-Derivate der Formel

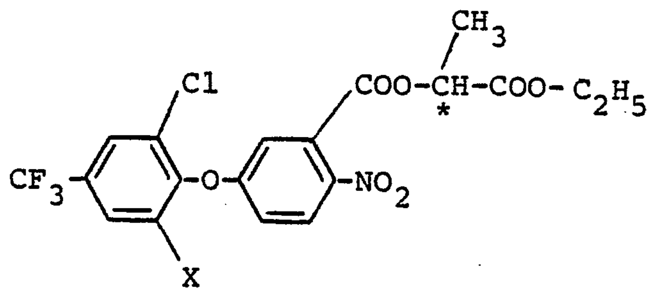

(I)

in welcher

X für Wasserstoff oder Chlor steht,

gefunden.

Weiterhin wurde gefunden, daß man die neuen linksdrehenden Enantiomeren der Phenoxybenzoesäure-Derivate der Formel (I) erhält, wenn man Phenoxybenzoesäurechloride der Formel

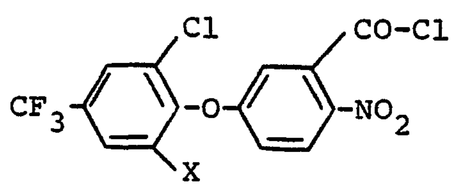

(II)

in welcher

X die oben angegebene Bedeutung hat,

mit dem rechtsdrehenden Enantiomeren des Milchsäureethylesters der Formel

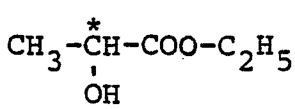

(III)

gegebenenfalls in Gegenwart eines Säureakzeptors sowie gegebenenfalls in Gegenwart eines Verdünnungsmittels umsetzt.

Schließlich wurde gefunden, daß sich die neuen linksdrehenden Enantiomeren der Phenoxybenzoesäure-Derivate der Formel (I) durch eine hervorragende herbizide Wirksamkeit auszeichnen.

Überraschenderweise besitzen die neuen linksdrehenden Enantiomeren der Phenoxybenzoesäure-Derivate der Formel (I) wesentlich bessere herbizide Eigenschaften als die entsprechenden racemischen Verbindungen, die aus dem Stand der Technik als hoch wirksame Herbizide bekannt sind.

Die erfindungsgemäßen Verbindungen sind durch die Formel (I) definiert. In dieser Formel ist das asymmetrische Kohlenstoffatom durch ein (*) gekennzeichnet.

Verwendet man bei dem erfindungsgemäßen Verfahren 5-(2,6-Dichlor-4-trifluormethyl-phenoxy)-2-nitrobenzoesäurechlorid und rechtsdrehendes Enantiomeres des Milchsäure-ethylesters, so kann der Verlauf der Umsetzung durch das folgende Formelschema wiedergegeben werden:

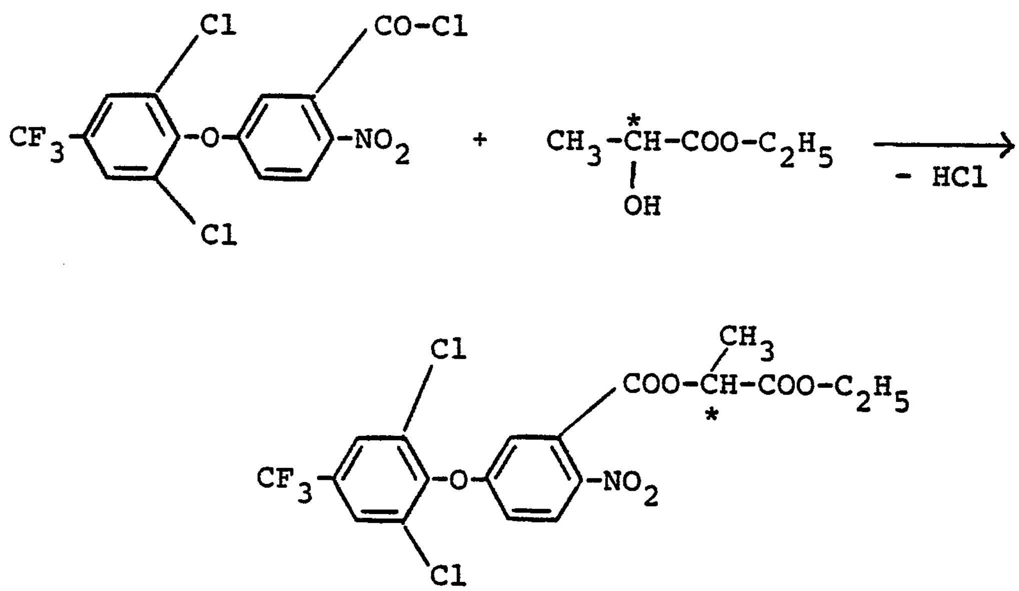


Die bei dem erfindungsgemäßen Verfahren als Ausgangsstoffe benötigten Phenoxybenzoesäurechloride sind durch die Formel (II) eindeutig definiert. In dieser Formel steht X für Wasserstoff oder Chlor.

Die Phenoxybenzoesäurechloride der Formel (II) sind bekannt (vgl. DE-OS 29 50 401).

Das bei dem erfindungsgemäßen Verfahren weiterhin als Ausgangsstoff benötigte rechtsdrehende Enantiomere des Milchsäure-ethylesters ist ebenfalls bekannt.

Das erfindungsgemäße Verfahren zur Herstellung der neuen linksdrehenden Enantiomeren der Phenoxybenzoesäure-Derivate der Formel (I) wird vorzugsweise unter Verwendung von Verdünnungsmitteln durchgeführt. Als derartige

Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die erfindungsgemäße Umsetzung wird außerdem vorzugsweise in Gegenwart eines Säurebindemittels vorgenommen. Als Säureakzeptoren können dabei alle üblichen Säurebindemittel eingesetzt werden. Vorzugsweise in Frage kommen Alkali-hydroxide, wie z.B. Natrium- und Kaliumhydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines größeren Bereiches variiert werden. Im allgemeinen arbeitet man zwischen -20 und +100°C, vorzugsweise zwischen 0 bis +50°C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in angenähert äquimolaren Mengen eingesetzt. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt.

Die anschließende Aufarbeitung erfolgt nach üblichen Methoden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den

folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Kon-

zentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan

oder Paraffine, z.B. Erdölfraktionen, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azofarbstoffe und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

Herstellungsbeispiele

Beispiel 1

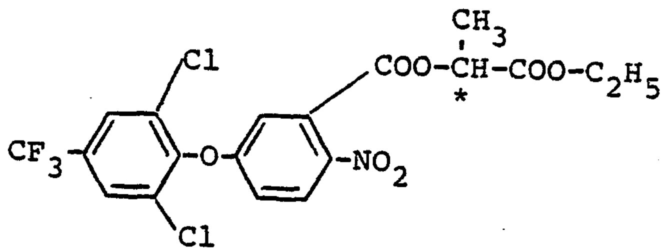

Zu einer Mischung aus 11,8 g (0,1 Mol) des rechtsdrehenden Enantiomeren des Milchsäureethylesters ($[\alpha]_D^{20}$ = +12°), 150 ml Toluol und 10,6 g (0,105 Mol) Triethylamin gibt man im Verlaufe von 35 Minuten bei einer Innentemperatur von 0 bis 5°C unter Rühren und Außenkühlung eine Lösung von 41,5 g (0,1 Mol) 5-(2,6-Dichlor-4-trifluormethylphenoxy)-2-nitro-benzoesäurechlorid in 150 ml Toluol. Nach der Zugabe wird die Reaktionsmischung 5,5 Stunden bei 20 - 25°C nachgerührt. Man gibt dann 100 ml Wasser zu der gerührten Lösung, trennt die Phasen und wäscht die organische Phase mit nochmals 100 ml Wasser. Dann wäscht man die organische Phase mit 150 ml 2 %iger wäßriger Natronlauge und anschließend so oft mit je 100 ml Wasser, bis das Waschwasser neutral ist. Die organische Phase wird getrocknet und unter vermindertem Druck vom Lösungsmittel befreit. Letzte Lösungsmittelreste werden durch Rotieren bei 70°C und 2 mbar (ca. 1 Stunde lang) entfernt.

Es hinterbleiben 45,7 g (92,1 % der Theorie) an linksdrehendem Enantiomeren des 5-(2,6-Dichlor-4-trifluormethyl-phenoxy)-2-nitro-benzoesäure-(1-ethoxycarbonyl-ethyl)-esters.

Drehwert: $[\alpha]_D^{20}$ = -15° (1-molare Lösung in Chloroform; Küvettenlänge = 10 cm)

Beispiel 2

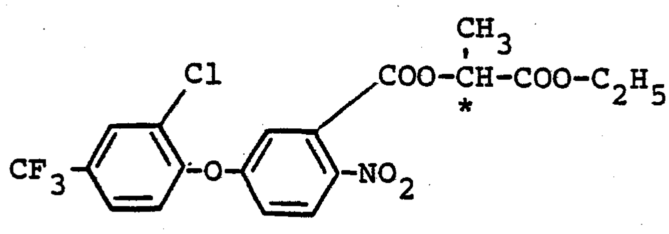

Zu einer Mischung aus 11,8 g (0,1 Mol) des rechtsdrehenden Enantiomeren des Milchsäureethylesters ($[\alpha]_D^{20}$ = +12°), 150 ml Toluol und 10,6 g Triethylamin (0,105 Mol) gibt man im Verlaufe von etwa 30 Minuten bei einer Innentemperatur von 0 bis 5°C unter Rühren und Außenkühlung eine Lösung von 38,0 g (0,1 Mol) 5-(2-Chlor-4-trifluormethyl-phenoxy)-2-nitro-benzoesäurechlorid in 150 ml Toluol. Nach der Zugabe wird die Reaktionsmischung 5,5 Stunden lang bei 20 bis 25°C nachgerührt. Man gibt dann 100 ml Wasser zu der gerührten Lösung, trennt die Phasen und wäscht die organische Phase mit nochmals 100 ml Wasser. Dann wäscht man die organische Phase mit 150 ml 2 %iger wäßriger Natronlauge und anschließend so lange mit je 100 ml Wasser, bis das Waschwasser neutral ist. Die organische Phase wird getrocknet und unter vermindertem Druck vom Lösungsmittel befreit. Letzte Lösungsmittelreste werden durch Rotieren bei 70°C und 2 mbar (ca. 1 Stunde lang) entfernt.
Es hinterbleiben 43,2 g (93,5 % der Theorie) an linksdrehendem Enantiomeren des 5-(2-Chlor-4-trifluormethyl-phenoxy)-2-nitro-benzoesäure-(1-ethoxycarbonyl-ethyl)-esters.

Drehwert: $[\alpha]_D^{20}$ = -9,8° (1-molare Lösung in Chloroform; Küvettenlänge =10 cm)

In den nachstehend beschriebenen Tests werden die folgenden Stoffe eingesetzt:

(A) = 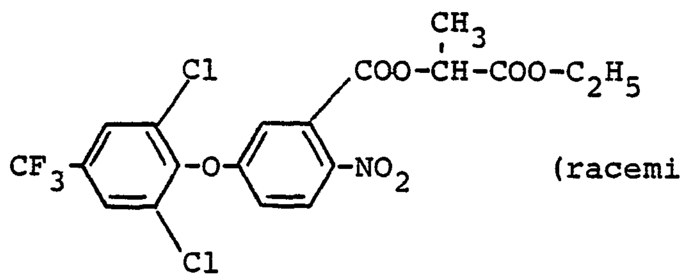 (racemisch)

(B) = 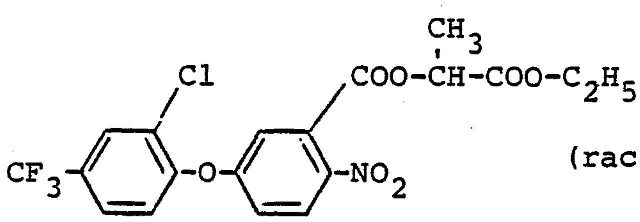 (racemisch)

(1) = 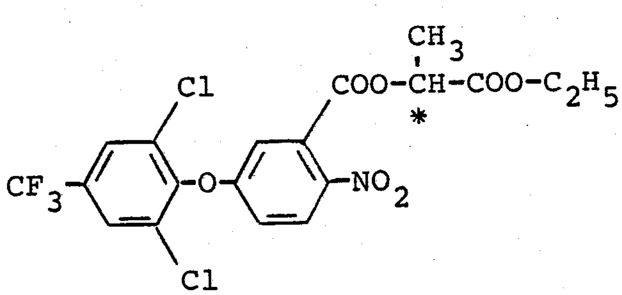 (linksdrehend)

(2) = 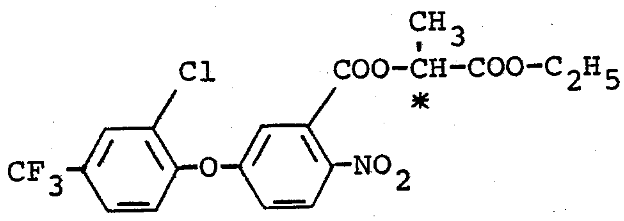 (linksdrehend)

Beispiel A

Pre-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe gemäß Beispielen 1 und 2 eine bessere herbizide Wirksamkeit als die entsprechenden racemischen Verbindungen (A) und (B).

## Tabelle A

**Pre-emergence-Test**

| Wirkstoff | Wirkstoff-aufwand (kg/ha) | Wirkung (in %) Dicotyledoneae *) | Monocotyledoneae **) | Rüben | Soja | Mais |
|---|---|---|---|---|---|---|
| (A) (bekannt) | 1 | 95,6 | 69,3 | 100 | 40 | 10 |
| (B) (bekannt) | 1 | 81,4 | 61,4 | 100 | 0 | 0 |
| (1) (erfindungsgemäß) | 0,6 | 92,7 | 79,3 | 100 | 10 | 10 |
| (2) (erfindungsgemäß) | 0,75 | 74,3 | 80,7 | 99 | 0 | 0 |

*) = durchschnittliche Wirkung bei mehreren Dicotylen

**) = durchschnittliche Wirkung bei mehreren Monocotylen

Beispiel B

Post-emergence-Test

Lösungsmittel: 5 Gewichtsteile Aceton
Emulgator : 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe gemäß Beispielen 1 und 2 eine bessere herbizide Wirksamkeit als die entsprechenden racemischen Verbindungen (A) und (B).

## Tabelle B

Post-emergence-Test

| Wirkstoff | Wirkstoff-aufwand (kg/ha) | Wirkung (in %) Dicotyledoneae *) | Monocotyledoneae **) | Rüben | Soja | Mais |
|---|---|---|---|---|---|---|
| (A) (bekannt) | 1 | 100 | 92 | 100 | 100 | 95 |
| (B) (bekannt) | 1 | 99,9 | 89,1 | 100 | 99 | 95 |
| (1) (erfindungsgemäß) | 0,6 | 99,3 | 81,1 | 100 | 100 | 80 |
| (2) (erfindungsgemäß) | 0,75 | 99,3 | 86,3 | 100 | 99 | 99 |

*) = durchschnittliche Wirkung bei mehreren Dicotylen
**) = durchschnittliche Wirkung bei mehreren Monocotylen

Patentansprüche

1. Linksdrehende Enantiomere der Phenoxybenzoesäure-Derivate der Formel

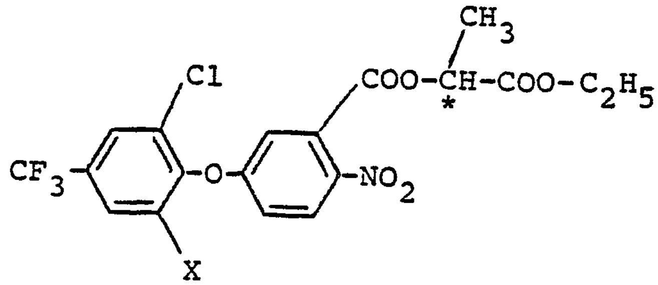

(I)

in welcher

X für Wasserstoff oder Chlor steht.

2. Verfahren zur Herstellung der linksdrehenden Enantiomeren der Phenoxybenzoesäure-Derivate der Formel

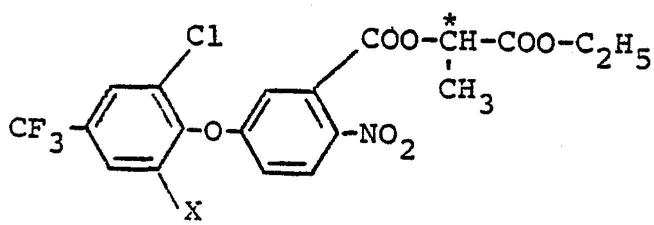

(I)

in welcher

X für Wasserstoff oder Chlor steht,

dadurch gekennzeichnet, daß man Phenoxybenzoesäurechloride der Formel

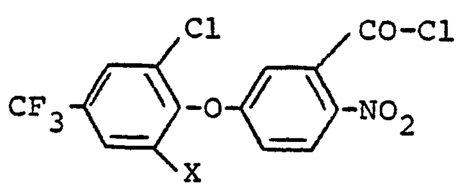

(II)

in welcher

X die oben angegebene Bedeutung hat,

mit dem rechtsdrehenden Enantiomeren des Milchsäureethylesters der Formel

$$CH_3-\underset{OH}{\overset{*}{C}}H-COO-C_2H_5 \qquad (III)$$

gegebenenfalls in Gegenwart eines Säureakzeptors sowie gegebenenfalls in Gegenwart eines Verdünnungsmittels umsetzt.

3. Herbizide Mittel, gekennzeichnet durch einen Gehalt an mindestens einem linksdrehenden Enantiomeren eines Phenoxybenzoesäure-Derivates der Formel (I).

4. Verwendung von linksdrehenden Enantiomeren der Phenoxybenzoesäure-Derivate der Formel (I) als Herbizide.

5. Verfahren zur Bekämpfung von Unkräutern, dadurch gekennzeichnet, daß man linksdrehende Enantiomere der Phenoxybenzoesäure-Derivate der Formel (I) auf die Unkräuter und/oder deren Lebensraum ausbringt.

6. Verfahren zur Herstellung von herbiziden Mitteln, dadurch gekennzeichnet, daß man linksdrehende Enantiomere der Phenoxybenzoesäure-Derivate der Formel (I) mit Streckmitteln und/oder oberflächenaktiven Stoffen vermischt.

Europäisches Patentamt

**EUROPÄISCHER RECHERCHENBERICHT**

0090204

Nummer der Anmeldung

EP 83 10 2204

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | | | C 07 C 79/46<br>C 07 C 76/00 |
| X | GB-A-2 058 055 (PPG INDUSTRIES) * Seite 1, Zeilen 1-27, Zeilen 38-56; Seite 2, Zeilen 14-35, 48-60 * | 1-6 | |
| | ----- | | |
| | | | **RECHERCHIERTE SACHGEBIETE (Int. Cl. 3)** |
| | | | C 07 C 79/00<br>C 07 C 76/00 |

Der vorliegende Recherchenbericht wurde für alle Patentansprüche erstellt.

| Recherchenort | Abschlußdatum der Recherche | Prüfer |
|---|---|---|
| DEN HAAG | 20-06-1983 | KINZINGER J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82